# EUROPEAN PATENT SPECIFICATION

(11) **EP 2 830 478 B1**
(45) Date of publication and mention of the grant of the patent: **21.08.2019**
(21) Application number: 13767441.2
(22) Date of filing: 28.02.2013
(51) Int. Cl.: A61B 1/00, A61B 17/00, A61M 25/00, F16L 1/00, A61B 1/12, A61B 1/005

(54) **INTEGRATED ENDOSCOPE IRRIGATION**
INTEGRIERTE ENDOSKOPBEWÄSSERUNG
IRRIGATION D'UN ENDOSCOPE INTÉGRÉ

(30) Priority: 27.03.2012 US 201261616097 P
(43) Date of publication of application: 04.02.2015
(73) Proprietor: MEDIGUS LTD., 8496500 Omer (IL)
(72) Inventor: GOVRIN, Amir, 5251511 Ramat Gan (IL); DLUGACH, Yekaterina, 8429905 Beer Sheva (IL); KOLATT, Tsafrir, 3095028 Zichron Ya'akov (IL)
(74) Representative: J A Kemp
(86) International application number: PCT/IL2013/050170
(87) International publication number: WO 2013/144944

(56) References cited:
- EP-B1- 1 794 486
- WO-A1-2004/026125
- GB-A- 2 268 883
- US-A- 4 867 138
- US-A- 4 950 278
- US-A1- 2008 091 074
- US-A1- 2010 331 658

## Description

### Field of the Invention

The invention is from the field of medical devices. More specifically, the invention is from the field of small diameter endoscopic devices.

### Background of the Invention

In various medical applications there are many advantages for using small diameter endoscopes and laparoscopes (collectively called endoscopes or endoscopic devices herein) having, for example, a maximum outer diameter of 3.2mm. Most importantly small diameter endoscopes can be introduced to desired locations within the body through small diameter natural orifices and lumens. Also in cases where introduction of the endoscope may be irritating, a small diameter endoscope may mitigate such phenomena. An example of a procedure in which small diameter endoscopes can be useful is transnasal endoscopy that in some cases may replace trans-oral endoscopy. Moreover, small diameter endoscopes may be introduced into body cavities by single incision laparoscopy, wherein the incision itself is of minimal dimensions.

By its nature, endoscopy entails incorporating many components adapted to perform various functions within a single elongated instrument. This fact sometimes conflicts with the desire for minimum diameter and size in general. Among these components are: vision mechanisms, e.g. video cameras; illumination means, e.g. optical fibers or LEDS; articulation means; tissue collection elements or other surgical tools; irrigation, insufflation, and more.

One of the ways to accommodate as many components and functions as possible to decrease the size of each individual component, e.g., using a smaller size camera or a smaller size fiber bundle. However, this is not always possible, there are limits to how much reduction can be achieved, and each size reduction has its cost in terms of performance and assembly complexity.

US 4,867,138 teaches a device comprising a sheath into which the insertion tube of a conventional endoscope is inserted. In various embodiments, the insertion tube of the endoscope fits tightly into the sheath in such a way that the inside of the sheath is divided into two separate fluid channels - one for feeding irrigation fluid to a treatment area and the other for draining excess irrigation fluid from the treatment area.

It is therefore a purpose of the present invention to reduce the diameter of endoscope devices.

It is another purpose of the present invention to provide a method of providing an endoscopic device with more components without increasing the cross section of the insertion tube.

Further purposes and advantages of this invention will appear as the description proceeds.

### Summary of the Invention

In a first aspect the invention is an endoscopic device as defined in present claim 1.

In embodiments of the invention the endoscopic device comprises at least one of:
(a) An articulation section located at the distal end of the insertion tube proximally to the distal tip. The articulation section is activated by cables or wires that pass through tubes that extend the length of the interior of the insertion tube from the handle section to the articulation section.
(b) An imaging device located in the distal tip. The imaging device is activated by power delivered to it and transmits images gathered by it via one or more cables, wires, or optical fibers that pass through one or more tubes that extend the length of the interior of the insertion tube from the handle section to the distal tip.
(c) Illumination means located in the distal tip. The illumination means are activated by wires or optical fibers that pass through one or more tubes that extend the length of the interior of the insertion tube from the handle section to the distal tip.
(d) One or more working channels that pass through the interior of the insertion tube from the handle section to the distal tip.
(e) One or more other components each of which is located at a location on the insertion tube or on the distal tip and is associated with a tube, wires, or cable that passes through the interior of the insertion tube from the handle section to the location.

In embodiments of the endoscopic device of the invention the handle section comprises components of an articulation mechanism including articulation cylinders that are sealed by gaskets, which are adapted to enable movement of the cables or wires that pass through the insertion tube to steer the articulation section without leakage of liquid or gas between the handle section and the insertion tube.

In embodiments of the endoscopic device of the invention the imaging device is a video camera.

In embodiments of the endoscopic device of the invention the components located at a location on the insertion tube or on the distal tip are selected from: lasers and radio frequency generators.

In a second aspect the invention is a method of providing an endoscopic device with reduced diameter as defined in present claim 6.

In embodiments of the method of the invention the liquid or gas that flows through the empty spaces between the plurality of tubes, wires, and cables that pass through the interior of the insertion tube from the handle section of the endoscope to the at least one nozzle is used for at least one of the following purposes: irrigation, insufflation, suction, cooling, heating, staining tissue, and therapy.

All the above and other characteristics and advantages of the invention will be further understood through the following illustrative and non-limitative description of embodiments thereof, with reference to the appended drawings.

The present invention is directed to endoscopic devices that typically comprise at least an imaging device, e.g. a video camera and accompanying illumination means in the distal tip and an articulation section that is activated by cables or wires that pass through the interior of the insertion tube from an articulation steering mechanism located in the handle section. Endoscopic devices frequently also comprise one or more working channels, through which surgical tools, e.g. forceps, and therapy devices, e.g. lasers or RF generators, can be introduced from the handle section to the space beyond the distal tip in order to collect samples or carry out various procedures. In addition there are frequently channels for other purposes, e.g. irrigation water or air to clean the camera lens, gas for insufflation, dye for staining tissue, liquid for cooling (or heating), and gas or liquid for therapy, e.g. delivery of drugs or medicine.

In present day endoscopic devices each of the working channels and the channels for irrigation and insufflation are small tubes that run through the insertion tube from handle to distal tip. As an alternative to having separate tubes for each component or function, endoscopes may comprise a multi-lumen tube which contains separate lumens for each component. Also the optical fibers or electric wires for the illumination means and the power and signal wires to and from the camera pass through similar tubes. All of these individual tubes and the articulation cables or wires are tightly packed into the interior of the insertion tube.

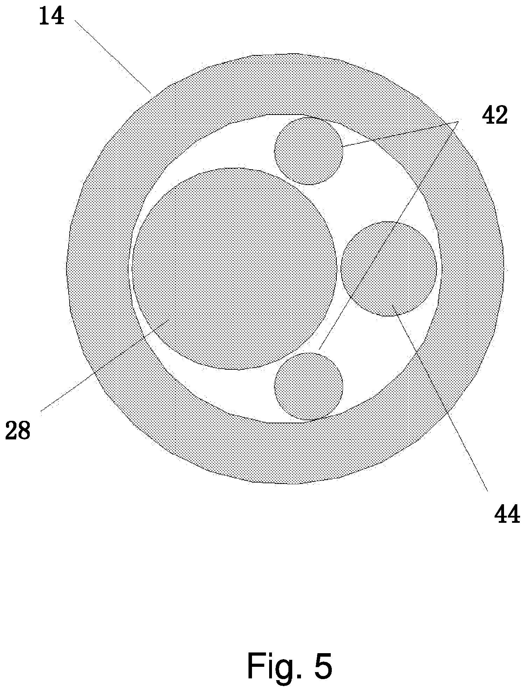

Fig. 5 schematically shows a cross-section of a typical insertion tube at a location between the handle and the articulation section. In the figure can be seen how the illumination fibers 28, camera cable 44, and articulation cables 42 are fit into the interior of insertion section 14. In this example, the minimum inner diameter of insertion tube 14 is determined by the diameters of the illumination fibers 28 and camera cable 44.

As can be seen from Fig. 5, packing a plurality of tubes having a circular cross-section into a larger cylindrical tube inevitable means that there will be empty spaces between the tubes that are not utilized. The current invention makes use of these spaces to allow fluid to flow from the handle of the endoscope to the distal tip. In this way it is not necessary to have a separate irrigation/insufflation/cooling/dye/therapy channel and as a result the overall diameter of the insertion tube can be reduced.

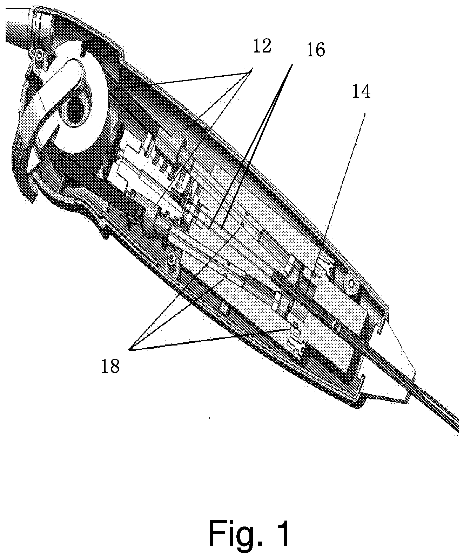

Fig. 1 to Fig. 4 illustrate one embodiment of an endoscope built according to the present invention.

Fig. 1 schematically shows the handle section of an embodiment of an endoscopic device with the cover partially removed to reveal some of the interior components. Shown in Fig. 1 are the articulation handle, articulation drum, and articulation cylinders of articulation mechanism 12; insertion tube 14; illumination fibers and power and signal wires 16; and gaskets 18.

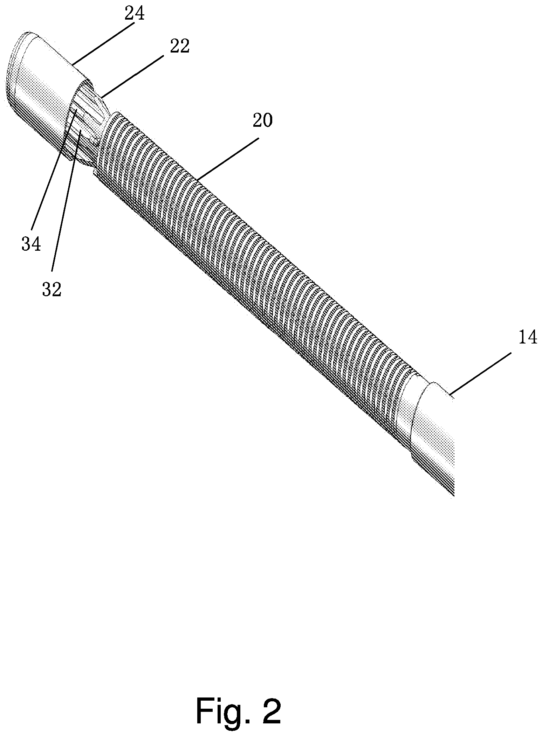

When the cover of the handle section is in place it presses against the gaskets 18 forming an air and water tight compartment in the volume defined by the gaskets. In particular the two small gaskets seal the articulation cylinders and enable movement of the steering cables or wires without leakage of fluid. An inlet port (not shown in the figure) allows water for irrigation or gas for insufflation to be introduced into this compartment. As will be described herein below, the water or gas flows out of the compartment in the handle into and through the insertion tube and exits the endoscope through a nozzle located on the distal tip. Fig. 2 schematically shows the distal end of an insertion tube 14 that is connected at its proximal end to the handle section shown in Fig.1. The sheath that covers the insertion tube has been removed to reveal articulation section 20 and distal tip 22. In contrast to conventional distal tips, which have smooth external surfaces that are shaped to minimize trauma as the endoscope is advanced to the site of the procedure, a pattern of alternating grooves 32 and lands 34 is created on the outer surface of distal tip 22. A cap 24, which fits tightly over the lands 34 provides the smooth outer surface. The tops of lands 34 are pressed against the inside wall of cap 24 forming hermetic seals that converts grooves 32 into closed channels through which water or gas flowing through the insertion tube can continue on its way to a nozzle on the distal end of the endoscope.

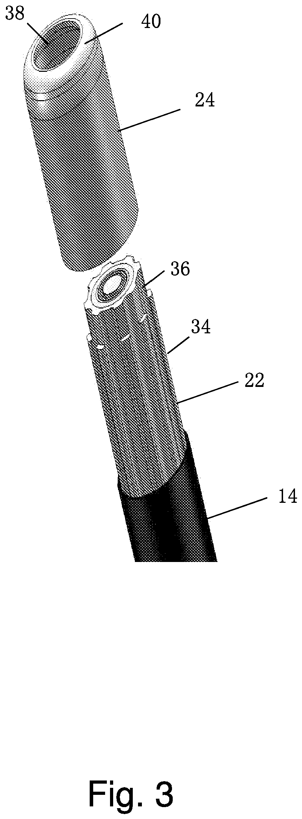

As shown in Fig. 3, a circular portion at the center of the distal surface of cylindrical cap 24 is removed leaving a hole 38 surrounded by an annular curved portion 40. As can also be seen in the same figure, a part 36 of the distal end of each land 34 is removed to form an annular space around the distal tip under curved portion 40 of cap 24.

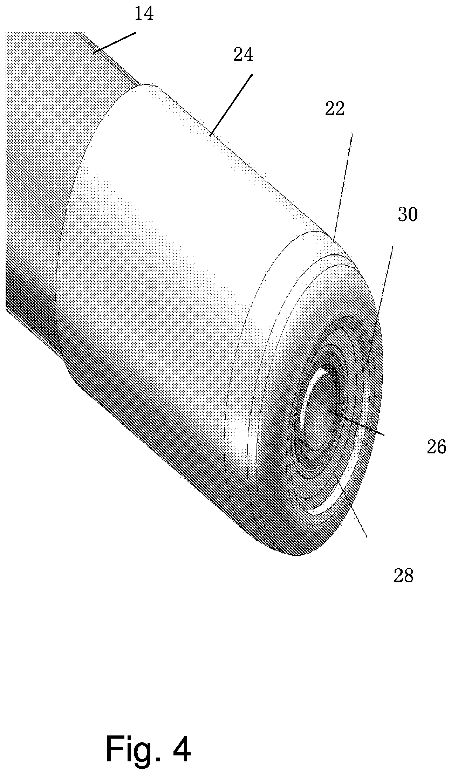

With the insertion section 14, including the articulation section, completely covered with the sheath and the cap 24 in place over the distal tip 22 as shown in Fig. 4, the inside of the endoscope is hermetically isolated from the outside with the exception of a small gap around the circumference of hole 38 in cap 24. The shape of curved surface 40 causes the gap to function as a circular nozzle 30. With the configuration described herein, the video camera 26 is located at the center of the distal tip surrounded by illumination fibers 28. Water or gas introduced into the interior of the handle enters the insertion tube 14 and flows through the empty spaces between the tubes and cables that pass through insertion tube 14 and articulation section 20. On reaching distal tip 22, the water or gas flows through grooves 32 and out of the distal end through circular nozzle 30. The components of the distal tip are configured such that water or gas exiting through circular nozzle 30 is sprayed over the illumination fibers 28 and objective lens of camera 26 keeping them clean.

It is noted that only a very basic embodiment of an endoscopic device has been described herein in order to illustrate the principle of the invention. In addition to a camera and illumination means, the endoscope may comprise one or more working channels, and other components located on the distal tip, e.g. ultrasound transducers. All of these require their own tube that must be integrated into the interior of the insertion tube. In these embodiments, eliminating the need for a separate irrigation and/or insufflation channel by utilizing the spaces between the other tubes for the passage of water or gas as taught by the present invention will play an important role in reducing the overall diameter of the insertion tube.

Although embodiments of the invention have been described by way of illustration, it will be understood that the invention may be carried out with many variations, modifications, and adaptations, without exceeding the scope of the claims.

## Claims

1. An endoscopic device that comprises a handle section, an insertion tube (14) connected to said handle section, a distal tip (22) at the distal end of said insertion tube (14), a plurality of tubes (28,42,44), wires, and cables (16) that pass through the interior of said insertion tube (14), and empty spaces between said plurality of tubes (28, 42,44), wires, and cables (16), wherein said empty spaces are configured to be utilized as a channel that enables liquid or gas to flow from said handle section to said distal tip (22);
**characterized in that** said distal tip (22) comprises a pattern of alternating grooves (32) and lands (34) on its outer circumferential surface and said 10 endoscopic device comprises a cap (24) comprising a circular hole (38) at the center of a distal surface of said cylindrical cap (24) surrounded by an annular curved portion (40), wherein said cap (24) is fitted tightly over said lands (34) thereby forming hermetic seals that convert said grooves (32) into closed channels between said lands (34) through which liquid or gas flowing through said empty spaces can continue on its way to a circular nozzle that is formed by a gap around the circumference of the circular hole (38) in said cap (24), wherein the shape of said annular curved portion (40) of said cap (24) causes the gap to function as said circular nozzle.

2. The endoscopic device of claim 1 comprising at least one of:
(a) an articulation section (20) located at the distal end of the insertion tube (14) proximally to the distal tip (22), said articulation section (20) activated by cables or wires that pass through tubes (42) that extend the length of the interior of said insertion tube (14) from the handle section to said articulation section (20);
(b) an imaging device (26) located in said distal tip (22), said imaging device (26) activated by power delivered to it and transmitting images gathered by it via one or more cables, wires, or optical fibers (16) that pass through one or more tubes (44) that extend the length of the interior of said insertion tube (14) from said handle section to said distal tip (22);
(c) illumination means (28) located in said distal tip (22), said illumination means activated by wires or optical fibers that pass through one or more tubes (28) that extend the length of the interior of said insertion tube (14) from said handle section to said distal tip (22);
(d) one or more working channels that pass through the interior of said insertion tube (14) from said handle section to said distal tip (22);
(e) one or more other components each of which is located at a location on said insertion tube or on said distal tip (22) and is associated with a tube, wires, or cable that passes through the interior of said insertion tube (14) from said handle section to said location.

3. The endoscopic device claim 2, wherein the handle section comprises components of an articulation mechanism (12) including articulation cylinders that are sealed by gaskets (18), which are adapted to enable movement of the cables or wires (42) that pass through the insertion tube (14) to steer the articulation section without leakage of liquid or gas between said handle section and said insertion tube (14).

4. The endoscopic device claim 2, wherein the imaging device is a video camera (26).

5. The endoscopic device of claim 2, wherein the components located at a location on the insertion tube (14) or on the distal tip (22) are selected from: lasers and radio frequency generators.

6. A method of providing an endoscopic device with reduced diameter, said endoscopic device comprising a handle section, an insertion tube (14) connected to said handle section, a distal tip (22) comprising a pattern of alternating grooves (32) and lands (34) on its outer surface at the distal end of said insertion tube (14), and a plurality of tubes (28,42,44), wires, and cables (16) that pass through the interior of said insertion tube (14); said method comprising utilizing empty spaces between said plurality of tubes (28,42,44), wires, and cables (16) as a liquid or gas channel that enables liquid or gas to flow from said handle section to said distal tip (22); said method **characterized by** fitting a cap (24) over said distal tip (22), said cap comprising a circular hole at the center of a distal surface of said cylindrical cap (24) surrounded by an annular curved portion (40), wherein said cap (24) fits tightly over said lands (34) thereby forming hermetic seals that convert said grooves (32) into closed channels between said lands (34) through which liquid or gas flowing through said empty spaces can continue on its way to a circular nozzle that is formed by a gap around the circumference of the circular hole (38) in said cap (24), wherein the shape of said annular curved portion (40) of said cap (24) causes the gap to function as said circular nozzle.

7. The method of claim 6, wherein liquid or gas that flows through the empty spaces between the plurality of tubes (24,42,44), wires, and cables (16) that pass through the interior of the insertion tube (14) from the handle section of said endoscope to said at least one nozzle (30) is used for at least one of the following purposes: irrigation, insufflation, suction, cooling, heating, staining tissue, and therapy.

## Patentansprüche

1. Endoskopische Vorrichtung, die einen Griffabschnitt, ein Einführungsrohr (14), das mit dem Griffabschnitt verbunden ist, eine distale Spitze (22) an dem distalen Ende des Einführungsrohrs (14), eine Vielzahl von Rohren (28, 42, 44), Drähten und Kabeln (16), die durch das Innere des Einführungsrohrs (14) verlaufen, und Leerräume zwischen der Vielzahl von Rohren (28, 42, 44), Drähten und Kabeln (16), wobei die Leerräume konfiguriert sind, als ein Kanal genutzt zu werden, der Flüssigkeit oder Gas ermöglicht, von dem Griffabschnitt zu der distalen Spitze (22) zu strömen;
**dadurch gekennzeichnet, dass** die distale Spitze (22) ein Muster aus sich abwechselnden Nuten (32) und Erhebungen (34) auf ihrer Außenumfangsfläche umfasst und die endoskopische Vorrichtung eine Kappe (24) umfasst, die ein kreisförmiges Loch (38) in der Mitte einer distalen Oberfläche der zylindrischen Kappe (24) umfasst, das von einem ringförmigen gekrümmten Abschnitt (40) umgeben ist, wobei die Kappe (24) eng über die Erhebungen (34) gesetzt ist, wodurch hermetische Dichtungen gebildet werden, die die Nuten (32) in geschlossene Kanäle zwischen den Erhebungen (34) umwandeln, durch die Flüssigkeit oder Gas, die/das durch die Leerräume strömt, ihren/seinen Weg zu einer kreisförmigen Düse fortsetzen kann, die durch einen Spalt um den Umfang des kreisförmigen Lochs (38) in der Kappe (24) gebildet ist, wobei die Form des ringförmigen gekrümmten Abschnitts (40) der Kappe (24) den Spalt dazu veranlasst, als die kreisförmige Düse zu fungieren.

2. Endoskopische Vorrichtung nach Anspruch 1, umfassend mindestens eines von Folgendem:
(a) einem Gelenkabschnitt (20), der sich an dem distalen Ende des Einführungsrohrs (14) proximal zu der distalen Spitze (22) befindet, wobei der Gelenkabschnitt (20) durch Kabel oder Drähte aktiviert wird, die durch Rohre (42) verlaufen, die sich über die Länge des Inneren des Einführungsrohrs (14) von dem Griffabschnitt zu dem Gelenkabschnitt (20) erstrecken;
(b) einer Bildgebungsvorrichtung (26), die sich in der distalen Spitze (22) befindet, wobei die Bildgebungsvorrichtung (26) durch an sie abgegebene Leistung aktiviert wird und Bilder überträgt, die durch sie über ein oder mehrere Kabel, Drähte oder Glasfasern (16) gesammelt werden, die durch ein oder mehrere Rohre (44) verlaufen, die sich über die Länge des Inneren des Einführungsrohrs (14) von dem Griffabschnitt zu der distalen Spitze (22) erstrecken;
(c) einem Beleuchtungsmittel (28), das sich in der distalen Spitze (22) befindet, wobei das Beleuchtungsmittel durch Drähte oder Glasfasern aktiviert wird, die durch ein oder mehrere Rohre (28) verlaufen, die sich über die Länge des Inneren des Einführungsrohrs (14) von dem Griffabschnitt zu der distalen Spitze (22) erstrecken;
(d) einem oder mehreren Arbeitskanälen, die durch das Innere des Einführungsrohrs (14) von dem Griffabschnitt zu der distalen Spitze (22) verlaufen;
(e) einer oder mehreren anderen Komponenten, die sich jeweils an einer Stelle auf dem Einführungsrohr oder auf der distalen Spitze (22) befinden und mit einem Rohr, Drähten oder einem Kabel in Verbindung sind, das durch das Innere des Einführungsrohrs (14) von dem Griffabschnitt zu der Stelle verläuft.

3. Endoskopische Vorrichtung Anspruch 2, wobei der Griffabschnitt Komponenten eines Gelenkmechanismus (12) umfasst, einschließlich Gelenkzylindern, die durch Dichtringe (18) abgedichtet sind, die angepasst sind, um Bewegung der Kabel oder Drähte (42), die durch das Einführungsrohr (14) verlaufen, zu ermöglichen, um den Gelenkabschnitt ohne Auslaufen von Flüssigkeit oder Gas zwischen dem Griffabschnitt und dem Einführungsrohr (14) zu lenken.

4. Endoskopische Vorrichtung Anspruch 2, wobei die Bildgebungsvorrichtung eine Videokamera (26) ist.

5. Endoskopische Vorrichtung nach Anspruch 2, wobei die Komponenten, die sich an einer Stelle auf dem Einführungsrohr (14) oder auf der distalen Spitze (22) befinden, aus Lasern und Hochfrequenzgeneratoren ausgewählt sind.

6. Verfahren eines Bereitstellens einer endoskopischen Vorrichtung mit reduziertem Durchmesser, wobei die endoskopische Vorrichtung Folgendes umfasst:
einen Griffabschnitt, ein Einführungsrohr (14), das mit dem Griffabschnitt verbunden ist, eine distale Spitze (22), die ein Muster aus sich abwechselnden Nuten (32) und Erhebungen (34) auf ihrer Außenfläche an dem distalen Ende des Einführungsrohrs (14) umfasst, und eine Vielzahl von Rohren (28, 42, 44), Drähten und Kabeln (16), die durch das Innere des Einführungsrohrs (14) verlaufen;
wobei das Verfahren ein Nutzen von Leerräumen zwischen der Vielzahl von Rohren (28, 42, 44), Drähten und Kabeln (16) als ein Flüssigkeits- oder Gaskanal umfasst, der Flüssigkeit oder Gas ermöglicht, von dem Griffabschnitt zu der distalen Spitze (22) zu strömen;
wobei das Verfahren **gekennzeichnet ist durch** ein Setzen einer Kappe (24) über die distale Spitze (22), wobei die Kappe ein kreisförmiges Loch in der Mitte einer distalen Oberfläche der zylindrischen Kappe (24) umfasst, das von einem ringförmigen gekrümmten Abschnitt (40) umgeben ist,
wobei die Kappe (24) eng über den Erhebungen (34) sitzt, wodurch hermetische Dichtungen gebildet werden, die die Nuten (32) in geschlossene Kanäle zwischen den Erhebungen (34) umwandeln, durch die Flüssigkeit oder Gas, die/das durch die Leerräume strömt, ihren/seinen Weg zu einer kreisförmigen Düse fortsetzen kann, die durch einen Spalt um den Umfang des kreisförmigen Lochs (38) in der Kappe (24) gebildet ist, wobei die Form des ringförmigen gekrümmten Abschnitts (40) der Kappe (24) den Spalt dazu veranlasst, als die kreisförmige Düse zu fungieren.

7. Verfahren nach Anspruch 6, wobei Flüssigkeit oder Gas, die/das durch die Leerräume zwischen der Vielzahl von Rohren (24, 42, 44), Drähten und Kabeln (16) strömt, die durch das Innere des Einführungsrohrs (14) von dem Griffabschnitt des Endoskops zu der mindestens einen Düse (30) verlaufen, für mindestens einen der folgenden Zwecke verwendet wird: Irrigation, Insufflation, Saugen, Kühlen, Erwärmen, Färben von Gewebe und Therapie.

## Revendications

1. Dispositif endoscopique qui comprend une section de manche, un tube d'insertion (14) raccordé à ladite section de tube, une pointe distale (22) à une extrémité distale dudit tube d'insertion (14), une pluralité de tubes (28, 42, 44), de fils et de câbles (16) qui passent à travers l'intérieur dudit tube d'insertion (14), et des espaces vides entre ladite pluralité de tubes (28, 42, 44), de fils et de câbles (16), lesdits espaces vides étant conçus pour être utilisés comme un canal qui permet l'écoulement de liquide ou de gaz de ladite section de manche à ladite pointe distale (22) ;
**caractérisé en ce que** ladite pointe distale (22) comprend un motif de rainures (32) et de plages (34) alternées sur sa surface circonférentielle externe et ledit dispositif endoscopique comprend un capuchon (24) comprenant un trou circulaire (38) au centre d'une surface distale dudit capuchon cylindrique (24) entouré par une partie incurvée annulaire (40), ledit capuchon (24) étant étroitement adapté sur lesdites plages (34) pour ainsi former un joint hermétique qui convertit lesdites rainures (32) en canaux fermés entre lesdites plages (34) à travers lesquels le liquide ou le gaz s'écoulant à travers lesdits espaces vides peut poursuivre son parcours jusqu'à une buse circulaire qui est formée par un espace autour de la circonférence du trou circulaire (38) dans ledit capuchon (24), la forme de ladite partie incurvée annulaire (40) dudit capuchon (24) amenant l'espace à fonctionner comme ladite buse circulaire.

2. Dispositif d'endoscopie de la revendication 1, comprenant au moins l'un des éléments suivant :
(a)une section d'articulation (20) située à l'extrémité distale du tube d'insertion (14) proximalement à la pointe distale (22), ladite section d'articulation (20) étant activée par des câbles ou fils qui passent à travers des tubes (42) qui s'étendant sur la longueur de l'intérieur dudit tube d'insertion (14) de la section de manche à ladite section d'articulation (20) ;
(b)un dispositif d'imagerie (26) situé dans ladite pointe distale (22), ledit dispositif d'imagerie (26) étant activé par de l'énergie délivrée à celui-ci et transmettant des images rassemblées par celui-ci via au moins un câble, fil ou fibre optique (16) qui passe à travers au moins un tube (44) qui s'étend sur la longueur de l'intérieur dudit tube d'insertion (14) de ladite section de manche à ladite pointe distale (22) ;
(c)un moyen d'éclairage (28) situé dans ladite pointe distale (22), ledit moyen d'éclairage étant activé par des fils ou fibres optiques qui passent à travers au moins un tube (28) qui s'étend sur la longueur de l'intérieur dudit tube d'insertion (14) de ladite section de manche à ladite pointe distale (22) ;
(d) au moins un canal de travail qui passe à travers l'intérieur dudit tube d'insertion (14) dudit manche à ladite pointe distale (22) ;
(e) au moins un autre composant qui est situé à un emplacement que ledit tube d'insertion ou sur ladite pointe distale (22) et est associé à un tube, des fils ou un câbles qui passent à travers l'intérieur dudit tube d'insertion (14) de ladite section de manche audit emplacement.

3. Dispositif endoscopique la revendication 2, dans lequel la section de manche comprend des composants d'un mécanisme d'articulation (12) comprenant des cylindres d'articulation qui sont scellés par des joints statiques (18), qui sont adaptés pour permettre un déplacement des câbles ou fils (42) qui passent à travers le tube d'insertion (14) pour diriger la section d'articulation sans fuite de liquide ou de gaz entre ladite section de manche et ledit tube d'insertion (14).

4. Dispositif endoscopique la revendication 2, dans lequel le dispositif d'imagerie est une caméra vidéo (26).

5. Dispositif endoscopique de la revendication 2, dans lequel les composants situés à un emplacement sur le tube d'insertion (14) ou sur la pointe distale (22) sont choisis parmi : des lasers et des générateurs de radiofréquence.

6. Procédé de fourniture d'un dispositif endoscopique de diamètre réduit, ledit dispositif endoscopique comprenant une section de manche, un tube d'insertion (14) raccordé à ladite section de tube, une pointe distale (22) comprenant un motif de rainures (32) et de plages (34) alternées sur sa surface externe à l'extrémité distale dudit tube d'insertion (14), et une pluralité de tubes (28, 42, 44), fils et câbles (16) qui passent à travers l'intérieur dudit tube d'insertion (14) ; ledit procédé comprenant l'utilisation d'espaces vides entre ladite pluralité de tubes (28, 42, 44), fils et câbles (16) en tant que canal de liquide ou de gaz permettant au liquide ou au gaz de s'écouler de ladite section de manche à ladite pointe distale (22) ;
ledit procédé étant **caractérisé par** l'adaptation d'un capuchon (24) sur ladite pointe distale (22), ledit capuchon comprenant un trou circulaire au centre d'une surface distale dudit capuchon cylindrique (24) entouré par une partie incurvée annulaire (40), ledit capuchon (24) s'adaptant étroitement que lesdites plages (34) pour ainsi former des joints hermétiques qui convertissent lesdites rainures en canaux fermé entre lesdites plages (34) à travers lesquels le liquide ou gaz s'écoulant à travers lesdits espaces vides peut poursuivre son parcours vers une buse circulaire qui est formée par un espace autour de la circonférence du trou circulaire (38) dans ledit capuchon (24), la forme de ladite partie incurvée annulaire (40) dudit capuchon (24) amenant l'espace à fonctionner comme ladite buse circulaire.

7. Procédé de la revendication 6, dans lequel le liquide ou gaz qui s'écoule à travers les espaces vides entre la pluralité de tubes (24, 42, 44), fils et câbles (16) qui passent à travers l'intérieur du tube d'insertion (14) de la section de manche dudit endoscope à ladite au moins une buse (30) est utilisé à au moins l'une des fins suivantes : irrigation, insufflation, aspiration, refroidissement, chauffage, coloration de tissu et thérapie.
